# EUROPÄISCHE PATENTSCHRIFT

(11) **EP 0 478 688 B1**
(45) Veröffentlichungstag und Bekanntmachung des Hinweises auf die Patenterteilung: **08.02.1995**
(21) Anmeldenummer: 90910725.2
(22) Anmeldetag: 26.07.1990
(51) Int. Cl.: C11D 1/66, A61K 7/16

(54) **PULVERFÖRMIGE ZUBEREITUNGEN OBERFLÄCHENAKTIVER ALKYLGLYKOSIDE**
POWDERED PREPARATIONS OF SURFACE ACTIVE ALKYLGLYCOSIDES
COMPOSITIONS PULVERULENTES D'ALKYLGLYCOSIDES SURFACTIFS

(30) Priorität: 04.08.1989 DE 3925858
(43) Veröffentlichungstag der Anmeldung: 08.04.1992
(73) Patentinhaber: Henkel Kommanditgesellschaft auf Aktien, 40191 Düsseldorf (DE)
(72) Erfinder: HILL, Karlheinz, Dr., D-4006 Erkrath (DE); FÖRG, Franz, Dr., D-4018 Langenfeld (DE); KÖRNER, Hermann, D-4000 Düsseldorf (DE); PENNINGER, Josef, Dr., D-4010 Hilden (DE)
(86) Internationale Anmeldenummer: EP9001226
(87) Internationale Veröffentlichungsnummer: WO9102046

(56) Entgegenhaltungen:
- EP-A- 0 304 627
- WO-A-87/02053
- US-A- 4 536 319

## Beschreibung

Die Erfindung betrifft pulverförmige Zubereitungen oberflächenaktiver Alkylglykoside mit guter Rieselfähigkeit sowie ein Verfahren zu ihrer Herstellung durch Trocknung einer wäßrigen Mischung von Alkylglykosid und inerten partikelförmigen Trägermaterialien.

Oberflächenaktive Alkylglykoside im Sinne der vorliegenden Erfindung sind die Reaktionsprodukte aus Zucker und aliphatischen primären Alkoholen mit vorzugsweise 8 - 22 Kohlenstoffatomen. Als Zuckerkomponenten, die im folgenden als Glykosen bezeichnet werden, kommen bevorzugt Glucose, daneben aber auch Fructose, Mannose, Galactose, Talose, Gulose, Allose, Altrose, Idose, Arabinose, Xylose, Lyxose, Ribose und Gemische in Frage.

Bevorzugt wegen der leichten Zugänglichkeit und der guten anwendungstechnischen Eigenschaften sind die Acetalisierungsprodukte der Glucose mit Fettalkoholen, die z. B. aus natürlichen Fetten und Ölen nach bekannten Verfahren erhältlich sind, d. h. mit linearen, primären, gesättigten und ungesättigten Alkoholen mit 8 - 22 Kohlenstoffatomen. Die Erfindung bezieht sich daher bevorzugt auf diese Typen der Alkylglykoside, im folgenden auch Alkylglucoside genannt.

Bei der Herstellung der Alkylglykoside nach den verschiedenen bekannten Verfahren z. B. nach US-A-3.839.318 oder EP 132 046 A1, werden diese mit unterschiedlichen Restmengen an nicht umgesetzten Ausgangsprodukten erhalten, von welchen insbesondere der Fettalkohol sich bei verschiedenen Anwendungen störend bemerkbar macht. Die rohen Acetalisierungsprodukte stellen feste, erstarrte Schmelzen dar, die sich bei Normaltemperatur nicht gießen und verteilen lassen.

Nach einem besonders bevorzugten Herstellungsverfahren wird Glucose mit Fettalkohol acetalisiert, wobei kontinuierlich Wasser bei reduziertem Druck aus dem Gemisch abdestilliert wird. Bei diesem Herstellungsverfahren, welches in der deutschen Patentanmeldungen P 38 33 780.0 näher beschrieben ist, fällt das rohe Alkylglucosid als erstarrte Schmelze an, die circa 63 Gew.-% Fettalkyl-Monoglucosid, 15 Gew.-% Fettalkyl-Diglucosid, 6 Gew.-% Fettalkyl-Triglucosid, 3 Gew.-% Alkyl-Tetraglucosid, 6 Gew.-% Polyglucose und 2 - 4 Gew.-% Rest-Fettalkohol enthält. Versuche, diese Schmelze durch Sprühkonfektionierung, durch Verschuppen oder Granulieren in ein bei Normaltemperatur rieselfähiges, partikelförmiges Produkt umzuwandeln, führten zu klebrigen und feuchtigkeitsempfindlichen Materialien. Der im Produkt noch enthaltene unumgesetzte Rest-Fettalkohol macht sich außerdem bei der Verwendung der Alkylglucoside in Mund- und Zahnreinigungsmitteln geschmacklich unangenehm bemerkbar. Mund- und Zahnpflegemittel stellen aber ein bevorzugtes Anwendungsgebiet für die Schleimhaut-verträglichen, oberflächenaktiven Alkylglykoside dar.

Aus EP-A-304 627 waren feste Konzentrate zur Herstellung von Mundwässern mit einem Gehalt an Biguaniden und Alkylglycosiden bekannt, die als Träger wasserlösliche Stoffe oder pyrogene Kieselsäure enthalten.

Es bestand daher die Aufgabe, oberflächenaktive Alkylglykoside in eine schütt- und rieselfähige Zubereitungsform zu überführen, die sich problemlos lagern, fördern, verteilen und in andere Produkte, insbesondere in Mund- und Zahnpflegemittel, problemlos einarbeiten läßt.

Gegenstand der Erfindung sind pulverförmige Zubereitungen oberflächenaktiver Alkylglykoside, die dadurch gekennzeichnet sind, daß sie 5 - 65 Gew.-% eines oberflächenaktiven Alkylglykosids und 35 - 95 Gew.-% eines inerten als Poliermittelkomponente in Zahnpasten geeigneten Trägers aus der Gruppe Kreide, abrasive Kieselsäuren einer mittleren Partikelgröße von 0,1 bis 10 »m, Dicalciumphosphat, Calciumpyrophosphat, unlösliches Natrium-Metaphosphat, Aluminiumoxid und Aluminiumoxidhydrat enthalten. Diese Zubereitungen Können durch Trocknung einer gießfähigen wäßrigen Mischung von rohem Alkylglykosid und Träger erhalten werden.

Unter einem rohen Alkylglykosid wird das nach dem bekannten Herstellverfahren anfallende Rohprodukt mit den verfahrensbedingten Nebenbestandteilen, insbesondere das nach dem oben beschriebenen Acetalisierungsverfahren erhältliche Reaktionsgemisch mit 2 - 5 Gew.-% an nicht umgesetzten Fettalkoholen verstanden.

Als Träger eignen sich solche Stoffe, die beim Einsatz in Mund- und Zahnpflegemittel, z. B. in Zahnpasten, nicht störend sind, z. B. weil sie bereits in anderer Funktion in solchen Zubereitungen erwünscht sind. Solche geeigneten Träger sind die bekannten Poliermittel-Komponenten, wie Kreide, Kieselsäuren, Dicalciumphosphat, Calciumpyrophosphat, unlösliches Natrium-Metaphosphat, Aluminiumoxid und Aluminiumoxidhydrate.

Besonders bevorzugt als Träger sind Kieselsäuren, die durch ihre hohe innere Oberfläche bereits in geringen Mengen ab etwa 0,5 Gewichtsteilen pro 1 Gewichtsteil Alkylpolyglykosid eine gute rieselfähige Zubereitung ergeben. Bei anderen Trägerstoffen, z. B. bei Kreide sollte das Gewichtsverhältnis von Träger zu Alkylglykosid wenigstens 1 : 1, besser noch 1 - 4 : 1 betragen, um anwendungstechnisch günstige und ausreichend lagerstabile Zubereitungen zu bekommen. Bevorzugte erfindungsgemäße Zubereitungen enthalten daher 20 - 50 Gew.-% eines oberflächenaktiven Alkylglykosids und 50 - 80 Gew.-% des inerten anorganischen Trägers.

Bevorzugt werden solche feinteiligen abrasiven Kieselsäuren als Träger verwendet, die auch bereits als Poliermittel für Zahnpasten in Gebrauch sind. Dies sind z. B. die Fällungskieselsäuren, wie sie aus DE-A-31 14 492 und DE-A-31 14 493 oder aus DE-A-24 46 038 bekannt und im Handel erhältlich sind, die wasserhaltigen Gelkieselsäuren, wie sie aus DE-A-27 04 504 und DE-A-28 53 647 bekannt sind und die Xerogelkieselsäuren, wie sie z. B. aus US-A-35 38 230 bekannt sind. Es können aber auch verdickende Kieselsäuren und pyrogene Kieselsäuren, z. B. Aerosil®, mitverwendet werden, wenn besonders niedrige Schüttdichten angestrebt werden.

Durch die Wahl der Art und Menge des Trägers und durch Kombinationen verschiedener Träger lassen sich die physikalischen und anwendungstechnischen Eigenschaften der erfindungsgemäßen pulverförmigen Zubereitungen in weiten Bereichen steuern. Für solche Zubereitungen, die als Zusatz zu Zahnpasten geeignet sein sollen und als Träger einen wasserunlöslichen Abrasivstoff enthalten, sollte die Partikelgröße des Trägers überwiegend unter 50 »m liegen und im Mittel bevorzugt 0,1 - 10 »m betragen.

Die Herstellung der erfindungsgemäßen Zubereitungen erfolgt dadurch, daß man 5 - 65 Gewichtsteile eines bei der technischen Herstellung anfallenden rohen Alkylglykosids mit 35 - 95 Gewichtsteilen des anorganischen Trägers und so viel Wasser mischt, daß eine bei einer Temperatur unterhalb + 80 °C noch fließfähige Mischung entsteht, und dieser Mischung das Wasser durch Verdampfung so weit entzieht, daß ein pulverförmiges, gut rieselfähiges Produkt entsteht. Man kann in diese Mischung auch die 30- bis 60prozentige wäßrige Paste des Alkylglucosids einsetzen, die nach der Bleichung des rohen Alkylglucosids nach dem Verfahren gemäß Deutscher Patentanmeldung P 38 33 780.0 erhalten wird. Es hat sich als günstig erwiesen, zur Herstellung dieser Mischung etwa 1,5 - 4 Gewichtsteile Wasser auf einen Gewichtsteil Feststoff einzusetzen. Diese Mischung stellt in der Regel eine bei Normaltemperatur wenig fließbare Paste (Slurry) dar, die beim Erwärmen auf Temperaturen bis 80 °C gut pumpfähig und versprühbar wird. In der Paste sind die wasserlöslichen Bestandteile weitgehend gelöst bzw. gequollen, die wasserunlöslichen Komponenten sind darin stabil dispergiert.

Der Entzug des Wassers aus dem Gemisch, d. h. die Trocknung der Paste bzw. des Slurry kann auf beliebige Weise erfolgen, z. B. auf Walzen- oder Bandtrocknern, in einem Wirbelbett aus fertiger Zubereitung oder durch Versprühen in einen Heißluftstrom. Das letztgenannte Verfahren, die sogenannte Sprühtrocknung, ist die bevorzugte Ausführungsform für die Trocknung der erfindungsgemäßen Zubereitungen. Der Entzug des Wassers erfolgt so weit, daß ein pulverförmiges, gut rieselfähiges Produkt entsteht. Dies ist in den meisten Fällen bei Wassergehalten von weniger als 1 Gew.-% der Fall. In Fällen, in denen der Träger einen Teil des Wassers als Kristallwasser bindet, bezieht sich dieser empfohlene Rest-Wassergehalt auf das freie, nicht im Kristallgitter gebundene Wasser. In Fällen, in welchen es bei der Trocknung zur Bildung von größeren Agglomeraten kommt, z. B. bei der Trocknung auf Walzen- und Bandtrocknern, kann es erforderlich sein, diese Agglomerate in einem nachgeschalteten Mahlprozeß zu desintegrieren. Bei Entfernung des Wassers durch Sprühtrocknung ist eine nachträgliche Desintegration der erfindungsgemäßen Zubereitungen nicht erforderlich. Zur Verwendung in Zahnpasten sollten die erfindungsgemäßen Zubereitungen keine Agglomerate mit Durchmessern über 200 »m enthalten, während die Primär-Korngröße im Mittel nicht über 50 »m liegen sollte.

Die erfindungsgemäßen Zubereitungen sind gut rieselfähig und behalten diese Rieselfähigkeit bei Lagerung auch in feuchter Luft bei. Ein weiterer Vorteil der pulverförmigen Zubereitungen liegt darin, daß durch das erfindungsgemäße Herstellverfahren der Gehalt an Rest-Fettalkohol stark verringert wird. Die Zubereitungen können daher auch zur Herstellung von Mund- und Zahnpflegemitteln verwendet werden, ohne daß es zu geschmacklichen Beeinträchtigungen kommt.

Die folgenden Beispiele sollen den Gegenstand der Erfindung näher erläutern ohne ihn hierauf zu beschränken.

### Beispiele

Aus den folgenden Rohstoffen wurden Mischungen gemäß Tabelle I hergestellt. Die Mischung Nr. 1 wurde in einem Trockenturm unter folgenden Sprühbedingungen versprüht:

| | | |
|---|---|---|
| Trocknungsluft: | Menge: | 600 m³/h |
| | Eintrittstemperatur: | 170 °C |
| | Austrittstemperatur: | 155 °C |
| Sprühdüse: | Zweistoffdüse, 1mm ⌀ | |
| Pastendruck: | 4,0 bar | |
| Zerstäubungsluft: | 4,0 bar | |

Dabei wurde ein rieselfähiges, feines weißes Pulver mit hervorragender Fließfähigkeit (Schüttwinkel 24,7 °) und einer Restfeuchte von 0,24 Gew.-% H₂O erhalten. Die Partikelgröße lag zwischen 1 und 180 »m mit Maxima bei 8 »m (Primärkorn) und 100 »m (Agglomerat). Die Mischungen Nr. 2 - 7 wurden im Laboratorium im Rotationsdampfer getrocknet. Dabei wurden ebenfalls gut rieselfähige, nicht klebende Pulver erhalten.

### Rohstoffe:

A) Alkylglucosid aus einem linearen C₁₂/C₁₄-Fettalkohol, hergestellt nach Deutscher Patentanmeldung P 38 33 780, Beispiel 3, enthaltend:
   3,0 Gew.-% restlichen Fettalkohol C₁₂/C₁₄
   62,8 Gew.-% Alkylmonoglucosid
   15,4 Gew.-% Alkyldiglucosid
   5,8 Gew.-% Alkyltriglucosid
   2,5 Gew.-% Alkyltetraglucosid
   1,1 Gew.-% Alkylpentaglucosid
   0,2 Gew.-% Alkylhexaglucosid
   6,0 Gew.-% Polyglucose
   unter 1,0 Gew.-% Glucose
   unter 2,0 Gew.-% Salze
B) Kreide (Schäfer Kreide N)
C) Fällungskieselsäure, Type Sident 12 SPLS, Degussa
D) Fällungskieselsäure, Type Sipernat 22 LS 22 SL, Degussa

**Tabelle I**

| Mischung Nr. | 1 | 2 | 3 | 4 | 5 |
|---|---|---|---|---|---|
| Alkylglucosid S | 10 | 15 | 10 | 5 | 15 |
| Sident^{(R)} 12 SPLS | 20 | 15 | - | - | - |
| Sipernat^{(R)} 22 LS | - | - | 10 | 10 | - |
| Schäfer Kreide N | - | - | - | - | 30 |
| Wasser | 70 | 70 | 80 | 85 | 65 |

Die Gehalte an restlichem Fettalkohol C₁₂/C₁₄ und der Rest-Wassergehalt lagen in allen durch Sprühtrocknung erhaltenen Pulvern unter 1 Gew.-%.

## Patentansprüche

1. Pulverförmige Zubereitungen oberflächenaktiver Alkylglykoside, dadurch gekennzeichnet, daß sie 5 - 65 Gew.-% eines oberflächenaktiven Alkylglykosids und 35 - 95 Gew.-% eines inerten, als Poliermittel-Komponente in Zahnpasten geeigneten Trägers aus der Gruppe Kreide, abrasive Kieselsäuren einer mittleren Partikelgröße von 0,1 bis 10 »m, Dicalciumphosphat, Calciumpyrophosphat, unlösliches Natrium-Metaphosphat, Aluminiumoxid und Aluminiumoxidhydrat enthalten.

2. Pulverförmige Zubereitungen nach Patentanspruch 1, dadurch gekennzeichnet, daß sie 20 - 50 Gew.-% eines oberflächenaktiven Alkylglykosids und 50 - 80 Gew.-% des inerten anorganischen Trägers enthalten.

3. Pulverförmige Zubereitungen nach einem der Patentansprüche 1 oder 2, dadurch gekennzeichnet, daß sie durch Trocknung einer gießfähigen, wäßrigen Mischung von rohe: Alkylglykosid und Träger erhalten werden.

4. Verfahren zur Herstellung pulverförmiger Zubereitungen von oberflächenaktiven Alkylglykosiden gemäß Anspruch 1, dadurch gekennzeichnet, daß man 10 - 60 Gewichtsteile eines bei der technischen Herstellung anfallenden rohen Alkylglykosids mit 50 - 90 Gewichtsteilen des inerten Trägers und mit so viel Wasser mischt, daß eine bei einer Temperatur unterhalb 80°C noch fließfähige Mischung entsteht, und dieser Mischung das Wasser durch Verdampfung so weit entzieht, daß ein pulverförmiges, rieselfähiges Produkt entsteht.

5. Verfahren nach Anspruch 4, dadurch gekennzeichnet, daß man zur Herstellung der Mischung 1,5 - 4 Gewichtsteile Wasser auf einen Gewichtsteil Feststoff einsetzt.

## Claims

1. Powder-form preparations of surface-active alkyl glycosides, characterized in that they contain 5 to 65% by weight of a surface-active alkyl glycoside and 35 to 95% by weight of an inert carrier - suitable as a polishing component in toothpastes - from the group consisting of chalk, abrasive silicas with an average particle size of 0.1 to 10»m, dicalcium phosphate, calcium pyrophosphate, insoluble sodium metaphosphate, aluminium oxide and hydrated aluminium oxide.

2. Powder-form preparations as claimed in claim 1, characterized in that they contain 20 to 50% by weight of a surface-active alkyl glycoside and 50 to 80% by weight of the inert inorganic carrier.

3. Powder-form preparations as claimed in claim 1 or 2, characterized in that they are obtained by drying of a pourable water-containing mixture of crude alkyl glycoside and carrier.

4. A process for the production of the powder-form preparations of surface-active alkyl glycosides claimed in claim 1, characterized in that 10 to 60 parts by weight of a crude alkyl glycoside from industrial-scale production are mixed with 50 to 90 parts by weight of the inert carrier and with water in such a quantity that a mixture still pourable at a temperature below 80°C is formed and water is removed from this mixture by evaporation to such an extent that a powder-form, free-flowing product is formed.

5. A process as claimed in claim 4, characterized in that 1.5 to 4 parts by weight of water to 1 part by weight of solid are used to prepare the mixture.

## Revendications

1. Préparations pulvérulentes d'alkylglycosides tensioactifs, caractérisées en ce qu'elles contiennent 5-65% en poids d'un alkylglycoside tensioactif et 35-95% en poids d'un support inerte approprié comme composant d'agent de polissage dans des dentifrices, du groupe de la craie, des acides siliciques abrasifs ayant une granulométrie moyenne de 0,1 à 10 »m, du phosphate dicalcique, du pyrophosphate de calcium, du métaphosphace de sodium insoluble, de l'oxyde d'aluminium et de l'oxyde-hydrate d'aluminium.

2. Préparations pulvérulentes selon la revendication 1, caractérisées en ce qu'elles contiennent 20-50% en poids d'un alkylglycoside tensioactif et 50-80% en poids du support inorganique inerte.

3. Préparations pulvérulentes selon l'une quelconque des revendications 1 ou 2, caractérisées en ce qu'on les obtient par séchage d'un mélange aqueux apte à l'écoulement d'alkylglycoside brut et d'un support.

4. Procédé pour la formulation de préparations pulvérulentes d'alkylglycosides tensioactifs selon la revendication 1, caractérisé en ce qu'on mélange 10-60 parties en poids d'un alkylglycoside brut que l'on obtient lors de la préparation technique avec 50-90 parties en poids du support inerte et avec une quantité d'eau telle que l'on obtient un mélange encore apte à l'écoulement à une température inférieure à 80°C, et on élimine l'eau de ce mélange par évaporation jusqu'à ce que l'on obtienne un produit pulvérulent apte au ruissellement.

5. Procédé selon la revendication 4, caractérisé en ce que, pour la préparation du mélange, on met en oeuvre 1,5-4 parties en poids d'eau pour 1 partie en poids de substance solide.
